# EUROPEAN PATENT SPECIFICATION

(11) **EP 4 384 009 B1**
(45) Date of publication and mention of the grant of the patent: **11.02.2026**
(21) Application number: 22765766.5
(22) Date of filing: 12.08.2022
(51) Int. Cl.: A01N 27/00, A01N 37/06, A01N 65/22, A01P 7/04, A61K 31/01, A61K 36/534, A01N 25/02

(54) **PEDICULICIDAL COMPOSITION**
PEDICULIZIDE ZUSAMMENSETZUNG
COMPOSITION PÉDICULICIDE

(30) Priority: 13.08.2021 BE 202105649
(43) Date of publication of application: 19.06.2024
(73) Proprietor: Oystershell NV, 9820 Merelbeke (BE)
(72) Inventor: EERTMANS, Frank, 9820 Merelbeke (BE); ROSSEL, Bart, 9820 Merelbeke (BE); VAN GANSE, Mike, 9820 Merelbeke (BE)
(74) Representative: Brantsandpatents bv
(86) International application number: PCT/EP2022/072707
(87) International publication number: WO 2023/017166

(56) References cited:
- WO-A1-2013/056044
- WO-A1-2019/008116
- MARTÍNEZ DE MURGUÍA FERNÁNDEZ LETICIA ET AL: "Effectiveness and tolerability of a squalane and dimethicone-based treatment for head lice", PARASITOLOGY RESEARCH, SPRINGER BERLIN HEIDELBERG, BERLIN/HEIDELBERG, vol. 120, no. 5, 2 April 2021 (2021-04-02), pages 1883 - 1890, XP037440043, ISSN: 0932-0113, [retrieved on 20210402], DOI: 10.1007/S00436-021-07113-Y

## Description

### FIELD OF THE INVENTION

The present invention relates to a composition for the treatment of infection by lice. In particular the present invention relates to bio-based, natural alternatives to the compositions for the treatment of infection by lice known in the art.

### BACKGROUND

There are three broad groups of pediculicidal compositions known in the art. The first relies on insecticides. There are however issues with respect to safety towards many insecticides when used frequently and directly on human skin.

The second group, which currently dominates the market, are compositions comprising silyl ether compounds and mineral oil-based products. Silyl ether compounds are known as efficient agents for the treatment of head lice infections or in the control of arthropods. The most prominent member of this class of compounds is polydimethylsiloxane (PDMS) or dimethicone. Dimethicone is a polymeric organosilicon compound which is a safe, effective, and physically acting alternative to insecticides for the treatment of head lice. In this respect, WO 2008/087148 describes foamable compositions for killing arthropods and their eggs, such as lice or ticks and nits. These formulations are effective. There are however issues with respect to ecology and sustainability. There are also some issues with skin irritation, sensitization, and allergic contact dermatitis.

The third group are bio-based compositions. US 2018/0177198 describes a pediculicide formulation based on Eucalyptus globulus essential oil, further comprising ethanol, isopropanol, and water. While the pediculicidal activity is attributed to a bio-based compound, the entire composition is not. Additionally, the composition has issues with skin irritation and sensitization due to the high alcohol content.

There are also compositions comprising both bio-based products and silyl ether compounds. US 8 574 641 describes a composition containing natural products, namely plant oils, but relies on the addition of (cosmetic) additives that are not biobased to obtain improved consumer satisfaction. These additives include diethylhexyl adipate, cyclopentasiloxane and dimethicone. It is clear that the composition as a whole is neither bio-based, nor fully biodegradable.

WO2013/056044 discloses a method of treating lice includes the steps of: (a) blending a treatment mixture; (b) applying the treatment mixture to a scalp or hair; and (c) applying a first shampoo to the scalp or the hair. The treatment mixture used in this method may contain olive oil and dimethicone.

WO2019/008116 teaches the use of squalane to kill lice.

There remains need in the art for an improved bio-based, natural composition for the effective treatment of infection by lice. The invention thereto aims to provide an ecological alternative for dimethicone-based products with high adulticidal activity. The invention aims to improve the activity, ease to use and customer satisfaction of present bio-based solutions. In particular, the invention aims to provide a composition which is safe for sensitive skin and eyes. The invention further aims to provide an ecological, bio-based, and biodegradable composition.

### SUMMARY OF THE INVENTION

The present invention and embodiments thereof serve to provide a solution to one or more of above-mentioned disadvantages. To this end, the present invention relates to a composition according to claim 1.

Preferred embodiments of the device are shown in any of the claims 2 to 13.

A specific preferred embodiment relates to an invention according to claim 3. The composition according to the present invention shows high adulticidal and ovicidal activity while being entirely bio-based. This is beneficial for consumers who prefer ecological or green products.

In a second aspect, the present invention relates to a use according to claim 14. Preferred embodiments of the method are shown in claim 15. The use as described herein is both effective and easy for the consumer. The use according to the present invention allows for easy application and in particular easy wash-out of the composition. It is mild and applicable for sensitive skin, as well as being safe for eyes. This makes the composition particularly suitable for using on children and adolescents, a group which frequently suffers from head lice.

The composition according to the present invention has the following benefits:
- It can be produced entirely from ECOCERT USDA bio-based materials, and is thus considered "green" and "organic",
- It does not comprise silicones,
- It has adulticidal activity comparable to dimethicone-based pediculicides,
- It has a fast method of action, lice are quickly immobilized thereby providing quick relief to a treated person,
- It has high adulticidal activity to pesticide resistant head lice, particularly headlice with knock down resistance (kdr)-type point mutations known to be resistant to insecticide-based treatments,
- It is mild for sensitive skin,
- It is safe for eyes,
- It provides beneficial esthetical effects,
- It provides a conditioning and moisturizing effect,
- It can easily be washed out,
- It does not contain a fragrance,
- It is suitable for pregnant and breast-feeding women,
- It's suitable for those older than 6 months.

### DETAILED DESCRIPTION OF THE INVENTION

The present invention concerns a composition for use in treatment of infection by lice. In particular the present invention relates to a green, bio-based or natural composition.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

A "green", "bio-based product" or "natural product" as used herein refers to any product that is composed of biological products, renewable agricultural materials or refined products using biological or renewable agricultural materials as intermediate feedstock. The definition of biological products thus includes products which are the result of biorefining. Biorefining is defined as a series of process steps or unit operations carried out on bio-based feedstocks. The process steps or unit operations include but are not limited to fermentation, hydrogenation and purification. In a preferred embodiment, the manufacturing, process steps and unit operations are adapted to be eco-friendly.

A "USDA biobased product" is a bio-based product as defined by the United States Secretary of Agriculture in the Farm Security and Rural Investment Act of 2002. A "USDA bio-based product" means a product determined by the Secretary to be a commercial or industrial product (other than food or feed) that is composed, in whole or in significant part, of biological products or renewable domestic agricultural materials (including plant, animal, and marine materials) or forestry materials or an intermediate feedstock. Biobased products also include biobased intermediate or 'upstream' materials such as bioresins or biopolymers, or the biobased/renewable chemicals used to create commercial, industrial, or consumer goods. The biobased content of a product is determined by the USDA Certified Biobased Product label. In a preferred embodiment, a bio-based product is certified by ECOCERT with the "Organic agriculture USA" certification. More preferably, a bio-based product is both ECOCERT certified and USDA biobased certified. ECOCERT Organic agriculture USA certification stands for :
- climate and environment protection
- conservation of soil fertility
- preservation of biodiversity
- respect of natural cycles and animal welfare
- absence of use of chemical and synthetic products
- absence of GMO
- transparent labelling for consumers

"Natural origin" as used herein and as defined by ECOCERT refers to products wherein all the ingredients are from natural origin except a restrictive approved ingredient list (including preservatives) authorized in small quantity. On average, ECOCERT certified products contain 99% ingredients of natural origin.

In a preferred embodiment, a bio-based product is COSMOS certified. The COSMOS-standard signature is a consumer guarantee for organic and natural cosmetics. The COSMOS-standard defines the criteria that companies must meet to ensure consumers that their products are genuine organic or natural cosmetics produced to the highest feasible sustainability practices. A care product is COSMOS ORGANIC certified only if at least 95% of the plants it contains are organic, and at least 20% of the organic ingredients are present in the total formula (10% for rinse-off products). Note that water or minerals are not regarded as "organic" for they are not from agriculture. Since water is a major component of numerous cosmetic formulas, it implies a dilution of the proportion of organic ingredients out of the total of the product. Thus a massage oil which does not contain water may display up to 100% organic ingredients.

The bio-based certifications as described herein, particularly "USDA bio-based", ECOCERT and COSMOS-standard are applicable to both finished consumer products as well as ingredients suitable for use in such products.

The expression "% by weight", "weight percent", "%wt" or "wt%", here and throughout the description unless otherwise defined, refers to the relative weight of the respective component based on the overall weight of the formulation. Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In a first aspect, the invention provides a composition comprising:
A. beeswax,
B. hemisqualane,
C. emulsifier, and
D. at least one vegetable oil, for use in treatment of infection by lice.

The composition is devoid of silicones.

The composition does not comprise dimethicone. Silicones, and dimethicone in particular, are commonly used as pediculicidal compounds. They generally have good adulticidal properties. However, consumers increasingly demand alternatives which do not comprise dimethicone, while still having similar pediculicidal activity. Common reasons include concerns of safety, concerns for negative effects on hair and skin and concerns for the ecological impact.

In a preferred embodiment of the invention, each constituent is a bio-based product. In a preferred embodiment of the invention, the composition is a bio-based product. In a more preferred embodiment, the composition contains at least 95% USDA biobased, more preferably 100% USDA bio-based ingredients. In a preferred embodiment, the composition is comprised of at least 95% ECOCERT certified ingredients, more preferably comprised of 100% ECOCERT certified ingredients, most preferably the composition itself is ECOCERT certified as Organic agriculture. In another preferred embodiment, the composition is comprised of at least 95% COSMOS-standard certified ingredients, more preferably comprised of 100% COSMOS-standard certified ingredients, most preferably the composition itself is COSMOS-standard certified. Most preferably, the product is 100% USDA bio-based, ECOCERT certified and COSMOS-standard certified. Bio-based products are more ecological. These certifications are considered an independent measurement of how organic or bio-based the product and its ingredients are.

In a preferred embodiment, the composition according to the present invention is biodegradable.

In a preferred embodiment, the composition comprises hemisqualane in an amount of at least 5% by weight of the composition, more preferably the composition comprises hemisqualane in an amount of at least 10% by weight of the composition, more preferably the composition comprises hemisqualane in an amount of at least 15% by weight of the composition, more preferably the composition comprises hemisqualane in an amount of at least 20% by weight of the composition, more preferably the composition comprises hemisqualane in an amount of at least 25% by weight of the composition, more preferably the composition comprises hemisqualane in an amount of at least 30% by weight of the composition, more preferably the composition comprises hemisqualane in an amount of at least 35% by weight of the composition, more preferably the composition comprises hemisqualane in an amount of at least 40% by weight of the composition, more preferably the composition comprises hemisqualane in an amount of at least 45% by weight of the composition. In a preferred embodiment, the composition comprises hemisqualane in an amount of at most 80% by weight of the composition, more preferably the composition comprises hemisqualane in an amount of at most 75% by weight of the composition, more preferably the composition comprises hemisqualane in an amount of at most 70% by weight of the composition, more preferably the composition comprises hemisqualane in an amount of at most 65% by weight of the composition, more preferably the composition comprises hemisqualane in an amount of at most 60% by weight of the composition, more preferably the composition comprises hemisqualane in an amount of at most 55% by weight of the composition, more preferably the composition comprises hemisqualane in an amount of at most 50% by weight of the composition. Hemisqualane is known to provide aesthetic benefits for hair and skin. In particular, it prevents moisture loss and nourishes hair and skin. It improves the shine of hair. Hemisqualane penetrates the hair shaft. Hemisqualane also strengthens hair and provides it with heat protection. It further enhances elasticity and prevents breakage. The inventors found that hemisqualane has high adulticidal and ovicidal activity when used in sufficiently high amounts. It is non-irritating, non-sensitizing and chemically inert. It is stable to heat, cold and UV. It is also biodegradable. Additionally, it is bio-based with consistent quality and pricing.

In a preferred embodiment, the hemisqualane is produced by fermentation and hydrogenation of sugar. More preferably, the sugar is sourced from sugarcane or sugar beet. Sugar is fermented, from which farnesene and other sesquiterpene compounds are separated and purified. Farnesene and possibly other sesquiterpene compounds are then hydrogenated to obtain hemisqualane. The obtained hemisqualane is a 100% fully saturated hydrocarbon. Sugarcane and sugar-beet based hemisqualane has consistent quality. It also has a stable and sustainable supply of feedstock. It is 100% USDA bio-based, ECOCERT certified and COSMOS-standard certified as an ingredient for consumer health and cosmetic products.

In another preferred embodiment, the composition comprises beeswax in an amount higher than 0.1% by weight, more preferably the composition comprises beeswax in an amount higher than 0.2% by weight, more preferably the composition comprises beeswax in an amount higher than 0.3% by weight, more preferably the composition comprises beeswax in an amount higher than 0.4% by weight, more preferably the composition comprises beeswax in an amount higher than 0.5% by weight, more preferably the composition comprises beeswax in an amount higher than 0.6% by weight, more preferably the composition comprises beeswax in an amount higher than 0.7% by weight, more preferably the composition comprises beeswax in an amount higher than 0.8% by weight, more preferably the composition comprises beeswax in an amount higher than 0.9% by weight, more preferably in an amount about 1.0% by weight. The percentages by weight are relative to the weight of the total composition. Beeswax a natural compound which is beneficial for its filmforming and antioxidant properties. It contributes to the adulticidal and especially ovicidal activity of the composition. It is also an emollient and provides a soothing effect to hair.

In another preferred embodiment, the composition comprises beeswax in an amount lower than 4.0% by weight, more preferably the composition comprises beeswax in an amount lower than 3.0% by weight, more preferably the composition comprises beeswax in an amount lower than 2.5% by weight, more preferably the composition comprises beeswax in an amount lower than 2.0% by weight, more preferably the composition comprises beeswax in an amount lower than 1.5% by weight. High amounts of beeswax are difficult to rinse or wash out.

In a preferred embodiment, the composition comprises beeswax in an amount of 0.2 to 4.0% by weight. More preferably, the composition comprises beeswax in an amount of 0.2 to 3.0% by weight, more preferably the composition comprises beeswax in an amount of 0.3 to 2.5% by weight, more preferably the composition comprises beeswax in an amount of 0.4 to 2.0% by weight, most preferably the composition comprises beeswax in an amount of 0.5 to 1.5% by weight. These ranges were found to be an optimum between providing the beneficial effects of beeswax, in particular ovicidal activity while remaining easy to wash-out of hair.

"Vegetable oil" according to the present invention refers to plant-sourced oils, including plant seed oils. In a preferred embodiment, the vegetable oil according to the present invention is chosen from the list of : evening primrose oil, black currant seed oil, borage oil, borage seed oil, safflower oil, safflower seed oil, sunflower oil, sunflower seed oil, sesame seed oil, peanut oil, walnut oil, almond oil, olive oil, olive seed oil, avocado oil, avocado seed oil, pumpkin seed oil, corn oil, cod liver oil, soy oil, soybean oil, coconut oil, palm oil, palm kernel oil, rapeseed oil, flaxseed (linseed) oil, cotton seed oil, tung oil, palmolein oil, mustard seed oil, oiticica oil and castor oil. In a more preferred embodiment, the vegetable oil according to the present invention comprises sunflower oil. However, some oils such as linseed, cottonseed and corn oil can undergo self-combustion. This problem was alleviated by using sunflower oil. Sunflower oil also improved manageability and frizz of the hair. It adds moisture, instant shine, and softness, providing a conditioning effect. This is nourishing and prevents breakage of the hair.

Plant oils can be extracted from plants or seeds using techniques that are known by those of skill in the art. In a preferred embodiment, the oils are extracted without hexane or the use of other chemical solvents. Not using hexane or solvents is ecologically beneficial. In a more preferred embodiment, the vegetable oil is "cold pressed" using an expeller-press method of extraction, which simply squeezes the oil out from the seed without the use of heat. This is ecologically beneficial. The preferred vegetable oils can all be extracted through cold pressing. Sunflower oil can be cold pressed to provide for a consistent high-quality vegetable oil with reliable and desirable properties.

As used herein, a "stabilizer" or "emulsifier" refers to any compound that can promote uniform separation of particles. Typical dispersing agents or emulsifiers are surface active agents or surfactants. As used herein, a "surface-active agent" or "surfactant" refers to any compound that reduces surface tension when dissolved in water or water solutions, or that reduces interfacial tension between two liquids, or between a liquid and a solid. Examples of surfactants include anionic, cationic, non-ionic and zwitterionic surfactants.

Emulsifiers that can be included in the compositions and formulations include, for example, ionic, non-ionic, zwitterionic and/or anionic surfactants. In some examples, the emulsifier is a surfactant that is anionic, such as sodium lauryl sulfate (USP) and its derivatives, alkyl sulfonate surfactants, a linear alkylbenzene sulfonic acid, a branched alkylbenzene sulfonic acid a C12 to C18 alkyl sulfate, C12-C18 alkyl alkoxy sulfate, C12-C18 alkyl methyl ester sulfonate, fatty soaps, alkyl sulfates, sulfated oils, ether sulfates, sulfonates, sulfosuccinates, sulfonated amides and isethionates; a zwitterionic surfactant; cationic surfactant, such as an alkylamine, an alkyl diamine, an alkyl polyamine, a mono- or di-quaternary ammonium salt, a monoalkoxylated amine, a dialkoxylated amine, a monoalkoxylated quaternary ammonium salt, a dialkoxylated quaternary ammonium salt, an etheramine, an amine oxide, an alkoxylated amine oxide and a fatty imidazoline, quaternary ammonium halides (such as cetyl pyridinium chloride); or a non-ionic surfactant, such as linear fatty alcohol ethoxylates or their polyoxyethylene condensation products (such as Spans and Tweens), alkyl arylpolyglycol ethers, polyethylene oxide esters of fatty acids, polyglycerol esters of fatty acids, polyoxyethylene sorbitan monolaurate, polyoxyethylene sorbitan mono- or tri-stearate, polyoxyethylene sorbitan monooleate, propylene glycol mono and diesters of fats and fatty acids, aryl sulfonates, sorbitan monostearate, poloxamer and its derivatives, medium chain triglyceride, caprylocaproyl macrogolglycerides, diethyleneglycol monoethyl ether, PEG-6 olive oil, PEG-6 peanut oil, PEG-6 hydrogenated palm kernel oil, propylene glycol dicaprylate/dicaprate, polysorbate, sorbitan esters, polyethoxylated castor oil, PEG-60 hydrogenated castor oil, PEG-40 hydrogenated castor oil, sodium lauryl glutamate, disodium cocoamphodiacetate, Polyoxyl 23 lauryl ester, an alkoxylated alcohol, a dialkoxylated alcohol, an alkoxylated dialkylphenol, an alkylpolyglycoside, an alkoxylated alkylphenol, an alkoxylated glycol, an alkoxylated mercaptan, an alkylamine salt, an alkyl quaternary amine salt, a glyceryl or polyglyceryl ester of a natural fatty acid, an alkoxylated glycol ester, an alkoxylated fatty acid, an alkoxylated alkanolamide, a polyalkoxylated silicone and an N-alkyl pyrrolidone, and combinations thereof. In a preferred embodiment, the emulsifier is sorbitan olivate; a sorbitan ester derived from sorbitol and the fatty acids of olive oil. It is a non-toxic, ECOCERT certified, COSMOS-standard certified, USDA bio-based, biodegradable emulsifier.

In a more preferred embodiment, the invention relates to a composition comprising:
A. beeswax, in an amount of 0.2 to 4.0 % by weight of the composition,
B. hemisqualane, in an amount of at least 10.0 % by weight of the composition,
C. emulsifier, and
D. At least one vegetable oil, preferably sunflower oil,
for use in treatment of infection by lice.

In a more preferred embodiment, the invention relates to a composition comprising:
A. beeswax, in an amount of 0.2 to 4.0 % by weight of the composition,
B. hemisqualane, in an amount of at least 30.0 % by weight of the composition,
C. emulsifier, and
D. at least one vegetable oil, preferably sunflower oil,
for use in treatment of infection by lice.

In a more preferred embodiment, the invention relates to a composition comprising:
A. beeswax, in an amount of 0.2 to 4.0 % by weight of the composition,
B. hemisqualane, in an amount of at least 40.0 % by weight of the composition,
C. emulsifier, and
D. At least one vegetable oil, preferably sunflower oil,
for use in treatment of infection by lice.

Resistance to pyrethrins or pyrethroids in insects is caused by multiple mechanisms, one of which is target site insensitivity, which is known as knockdown resistance or *kdr.* The *kdr* phenotype (recalcitrant to knockdown), a heritable trait associated with nerve insensitivity to DDT, the pyrethrins, and pyrethroids, and is one of the most common mechanisms of resistance in insects against these insecticides. While the presence of *kdr*-type mutations alone may not directly predict clinical failure, their increasing frequency in louse populations coincides with reports of product failures. The inventors have surprisingly found that the present compositions and treatments are highly effective against human head lice with knockdown resistance phenotype (referred to as "resistant headlice").

In a preferred embodiment, the invention provides a preventive effect. An issue is that infections or infestations of arthropods, in particular lice are often spread through gatherings or groups of humans or animals (e.g. schools, youth movements, ...). While various agents that effectively kill lice and nits are known in the art, few agents that prevent infection or infestation from the environment are known. This leads to an infection or infestation often persisting for several weeks until the infection or infestation of arthropods has been removed from (almost) all humans or animals in the school or gathering. The inventors surprisingly found that hair conditioning agents, perfumes or a combination thereof repelled oviposition and generally prevented infestation or infection by arthropods. This is very desirable to avoid the spreading, infection or potential re-infection or infestation of arthropods in groups of humans or animals.

In a second aspect, the invention relates to the use of a composition according to the first aspect for the treatment of infection by lice. In a preferred embodiment, the invention relates to the to the use of a composition according to the first aspect for the treatment of infection by lice in humans, more preferably head lice in humans.

In a preferred embodiment of the invention, the invention provides a fast method of action. In particular it is desirable to provide high adulticidal activity quickly after the treatment. In a preferred embodiment, the composition is suitable for quick relief. In a further preferred embodiment, a 10 minute treatment according to the second aspect and / or treatment using the composition of the first aspect immobilizes at least 80% of lice, more preferably at least 85% of lice, more preferably at least 90% of lice, more preferably at least 95% of lice, more preferably at least 96% of head lice, more preferably at least 97% of head lice, more preferably at least 98% of head lice within 15 minutes after the treatment, more preferably within 10 minutes after the treatment, more preferably within 5 minutes after the treatment, more preferably within 3 minutes after the treatment.

Head lice most commonly occur in children and adolescents. The composition of the present invention can advantageously be used safely, easily and effectively to treat children and adolescents. The composition is an effective adulticidal and ovicidal. But its added benefit is being mild, well suited for sensitive skin, safe for eyes, non-irritating and non-sensitizing. It also provides beneficial esthetic effects to hair and skin, such as conditioning and moisturizing.

In a preferred embodiment, the use of the composition for treating hair for instance comprises the steps of: (a) applying an effective amount of the composition according to the present invention to the wet or dry hair and the scalp, (b) working the composition according to the present invention in contact with the hair and scalp, especially to the region behind the ears and the hairline at the back of the head, (c) leaving the composition according to the present invention on the hair for a suitable period of time to allow killing to occur and (d) rinsing the composition according to the present invention from the hair using water alone or water and soap. Application of the composition according to the present invention to the hair typically includes working the composition according to the present invention through the hair, generally with the hands and fingers. The composition according to the present invention is left into contact with the hair, e.g. for about 5 or 10 minutes. The composition according to the present invention is then rinsed from the hair with water and optionally soap (e.g. a shampoo).

In a preferred embodiment, the treatment is carried out twice with an intermediate waiting period of about 1 week. The treatment after one week aims to kill the hatched lice that were not killed in the first treatment.

The invention is further described by the following non-limiting examples which further illustrate the invention, and are not intended to, nor should they be interpreted to, limit the scope of the invention.

### EXAMPLES AND/OR DESCRIPTION OF FIGURES

6 different compositions were tested for consumer acceptance, safety (including ISO10993 standard for biocompatibility of medical devices to manage biological risk) and pediculicidal activity.

### Comparative example 1

Comparative example 1 is a composition consisting of 96 wt.% cottonseed oil and 4 wt.% beeswax. The composition was tested for consumer acceptance, safety and pediculicidal activity.

ISO10993 tests showed no cytotoxicity, no sensitization and no skin irritation. Patch tests showed no irritation in normal and sensitive skin.

Pediculicidal activity was tested on head lice. The composition was left in contact with the head lice throughout a 5 minute and 15-minute treatment, respectively. This resulted in 98.7% (5 min) to 100% (15 min) lice mortality 3 hours after the treatment. Both treatments thus showed very high adulticidal activity.

The composition failed the consumer acceptance tests: the composition was greasy and very difficult to wash out of hair.

### Comparative example 2

Comparative example 2 is a composition consisting of 98 wt.% cottonseed oil and 2 wt.% beeswax. The composition was tested for consumer acceptance, safety and pediculicidal activity.

Pediculicidal activity was tested on head lice and their ova. The composition was left in contact with the head lice and the ova throughout a 15-minute treatment. The fifteen-minute treatment showed 19% lice mortality 3 hours after the treatment. The fifteen-minute treatment showed 70.4% ovicidal activity. The adulticidal activity was clearly lackluster.

Consumer acceptance tests showed improved, but not perfect results. The composition was easier to rinse out of hair, but further improvements were desired.

### Comparative Example 3

Comparative example 3 is a composition consisting of 88 wt.% cottonseed oil, 2 wt.% beeswax, and 10% hemisqualane. The composition was tested for consumer acceptance, safety and pediculicidal activity.

ISO10993 tests showed no cytotoxicity, no sensitization and no skin irritation. Patch tests showed no irritation in normal and sensitive skin. However, this mixture was found to be prone to spontaneous combustion due to the presence of cottonseed oil.

Pediculicidal activity was tested on head lice. The composition was left in contact with the head lice throughout a 5-minute treatment. The five-minute treatment showed 50% lice mortality 3 hours after the treatment. Lice mortality 80% 18 hours after the treatment.

### Comparative example 4

Comparative example 4 is a composition consisting of 48 wt.% sunflower oil, 1 wt.% beeswax, 50% hemisqualane, 0.75% lavender extract and 0.25% peppermint oil. The composition was tested for consumer acceptance, safety and pediculicidal activity.

Pediculicidal activity was tested on head lice and their ova. The composition was left in contact with the head lice and their ova throughout a 5-minute treatment. The five-minute treatment showed 97% lice mortality 3 hours after the treatment and 100% lice mortality 18 hours after the treatment. The five-minute treatment showed 77% ovicidal activity. Additionally, it was noted that the majority of head lice became immobilized and likely died immediately following treatment. This quick death of head lice is desirable and common for dimethicone treatments..

### Example 5

Example 5 is a composition consisting of 44% sunflower oil, 50% hemisqualane, 1% beeswax and 4% sorbitan olivate. The composition was tested for consumer acceptance, safety and pediculicidal activity.

ISO10993 tests showed no cytotoxicity.

Pediculicidal activity was tested on head lice as well as their ova. The composition was left in contact with the head lice and their ova throughout a 10-minute treatment, showing 100% lice mortality 3 hours after the treatment. The ten-minute treatment showed 97% ovicidal activity. A fifteen-minute treatment showed 100% ovicidal activity. As in example 4, it was noted that the majority of head lice became mobilized and likely died immediately following treatment. The desired dimethicone effect was obtained. The treatment showed improved ovicidal activity.

The consumer acceptance was tested. Consumer acceptance was high. The composition was easily removed by rinsing with water.

### Comparative example 6

Pediculicidal activity was tested on insecticide-resistant head lice (Pediculus humanus capitis). Three compositions were evaluated:
- the composition of example 5;
- comparative example 6: a commercially available, permethrin-based (1%) pediculicide; and
- negative control (water).

Efficacy was determined based on the number of dead lice (defined as no general movement and absence of gastrointestinal motility). Freshly collected and viable head lice were transferred to human Petri dishes, lined with corduroy fabric pre- and post-treatment. Dose was 2 ml, treatment duration was 10 min. Examples 5 and 6 were tested in parallel with a water control (negative control). Post treatment head lice were observed every 5 minutes for one hour and consequently every hour for a total of four hours, with final evaluation 24hr post-treatment.

Lice were categorized as follows:
1) Fully active: mobile lice showing ability to grab on and cling to hair
2) Partially active: partial mobile lice showing movement in appendages and gut mobility. These lice cannot grab on hair anymore.
3) Gut mobility: lice with only gut mobility present
4) Inactive: totally inactive lice, no appendage movement, no gut mobility nor ability to grab on or cling to hair - dead.

The efficacy of example 5 against crawling stages (3^{O} instar nymphs and adults) of the head lice, Pediculus humanus capitis, with a dose of 2 ml, can be described as follows:
- Five minutes post-treatment, a high number (97.33%) of head lice were inactive, whereas 2.67% of the lice only showed gut mobility.
- The high % of mortality remained constant over the next 4 hours post treatment, with only a very low % of head lice being fully active or being knockdown (1.33-2.67%), respectively.
- At the end of the study (24h), all head lice were dead (100% mortality). Data clearly indicate that the composition of example 5 quickly kills resistant head lice with high efficacy.

In contrast, in comparative example 6, the following observations were made:
- 5 minutes post-treatment, only 34.67% of the treated lice were inactive. 18.67% were partially active or only showed gut mobility, whereas 46.67% remained fully active.
- Between 5 minutes and 2 hours post-treatment, the number of active lice increased to 61,33%, whereas only 2,67% to 6.67% of the head lice were inactive. The remaining lice were either partially inactive or only showed gut mobility.
- 4 hours post treatment, mortality remained limited to 29.33% (and 46.67% active lice).
- Finally, after 24h, lice mortality increased.

These data indicate that 1% permethrin does not act fast on head lice, in contrast to the composition according to example 5. In the negative control (water), 100% of the lice were fully active 5 min to 24h post treatment.

A genetic analysis was performed on treated lice (both test groups) to assess the presence of knockdown gene resistance (kdr) gene mutations. The latter gene mutations are contributing to conventional pesticide resistance (including pyrethroids). The following mutations were analyzed: M815I, T917I, and L920F, respectively. Said kdr point mutations were detected in the 3 target genes in all treatment groups, confirming that confirms that head lice, used in all treatment groups, are resistant to conventional pesticides.

Concluding, treatment (10 min) with the composition according to example 5 resulted in fast and high mortality of the head lice. At study end, all head lice showed 100% mortality. In contrast, in the treatment with comparative example 6, the number of inactive head lice was low (varying between 2.67% and 34.67%). During the 4-hour observation period following treatment, 46.67% to 68% of the head lice remained fully active. Only 24 hours post treatment, all lice were dead. In the negative control group, none of the lice died during the observation period. This data clearly indicates that a composition according to present invention is able to quickly kill resistant head lice, whereas conventional commercial products showed rather weak performance

## Claims

1. Composition, for use in treatment of infection by lice, comprising:
A. beeswax,
B. hemisqualane,
C. an emulsifier, and
D. at least one vegetable oil,
wherein said composition is devoid of silicones.

2. Composition for use according to claim 1, comprising beeswax in an amount of 0.2 to 4.0 % by weight of the composition.

3. Composition for use according to any of the preceding claims 1 to 2, comprising hemisqualane in an amount of at least 10 % by weight of the composition.

4. Composition for use according to any of the preceding claims 1 to 3, comprising hemisqualane in an amount of at least 40% by weight of the composition.

5. Composition for use according to any of the preceding claims 1 to 4, wherein the vegetable oil is sunflower oil.

6. Composition for use according to any of the preceding claims 1 to 5, wherein said emulsifier comprises sorbitan esters.

7. Composition for use according to any of the preceding claims 1 to 6, wherein said emulsifier comprises sorbitan olivate.

8. Composition for use according to any of the preceding claims 1 to 7, comprising:
A. beeswax, in an amount of 0.2 to 4.0 % by weight of the composition,
B. hemisqualane, in an amount of at least 10.0 % by weight of the composition,
C. an emulsifier, and
D. a vegetable oil, preferably sunflower oil.

## Patentansprüche

1. Zusammensetzung zur Verwendung bei der Behandlung einer Infektion durch Läuse, umfassend:
A. Bienenwachs,
B. Hemisqualan,
C. einen Emulgator, und
D. mindestens ein Pflanzenöl,
wobei die Zusammensetzung frei von Silikonen ist.

2. Zusammensetzung zur Verwendung nach Anspruch 1, Bienenwachs in einer Menge von 0,2 bis 4,0 Gew.-% der Zusammensetzung umfassend.

3. Zusammensetzung zur Verwendung nach einem der Ansprüche 1 bis 2, Hemisqualan in einer Menge von mindestens 10 Gew.-% der Zusammensetzung umfassend.

4. Zusammensetzung zur Verwendung nach einem der Ansprüche 1 bis 3, Hemisqualan in einer Menge von mindestens 40 Gew.-% der Zusammensetzung umfassend.

5. Zusammensetzung zur Verwendung nach einem der Ansprüche 1 bis 4, wobei das Pflanzenöl Sonnenblumenöl ist.

6. Zusammensetzung zur Verwendung nach einem der Ansprüche 1 bis 5, wobei der Emulgator Sorbitanester umfasst.

7. Zusammensetzung zur Verwendung nach einem der Ansprüche 1 bis 6, wobei der Emulgator Sorbitanolivat umfasst.

8. Zusammensetzung zur Verwendung nach einem der Ansprüche 1 bis 7, umfassend:
A. Bienenwachs in einer Menge von 0,2 bis 4,0 Gew.-% der Zusammensetzung,
B. Hemisqualan in einer Menge von mindestens 10,0 Gew.-% der Zusammensetzung,
C. einen Emulgator, und
D. mindestens ein Pflanzenöl, vorzugsweise Sonnenblumenöl.

## Revendications

1. Composition, destinée à être utilisée dans le traitement de l'infection par les poux, comprenant :
A. cire d'abeille,
B. hémisqualane,
C. un émulsifiant, et
D. au moins une huile végétale,
dans laquelle ladite composition est dépourvue de silicones.

2. Composition pour utilisation selon la revendication 1, comprenant de la cire d'abeille en une quantité de 0,2 à 4,0 % en poids de la composition.

3. Composition pour utilisation selon l'une quelconque des revendications précédentes 1 à 2, comprenant de l'hémisqualane en une quantité d'au moins 10 % en poids de la composition.

4. Composition pour utilisation selon l'une quelconque des revendications précédentes 1 à 3, comprenant de l'hémisqualane en une quantité d'au moins 40 % en poids de la composition.

5. Composition pour utilisation selon l'une quelconque des revendications précédentes 1 à 4, dans laquelle l'huile végétale est l'huile de tournesol.

6. Composition pour utilisation selon l'une quelconque des revendications précédentes 1 à 5, dans laquelle ledit émulsifiant comprend des esters de sorbitane.

7. Composition pour utilisation selon l'une quelconque des revendications précédentes 1 à 6, dans laquelle ledit émulsifiant comprend l'olivate de sorbitane.

8. Composition pour utilisation selon l'une quelconque des revendications précédentes 1 à 7, comprenant :
A. cire d'abeille, en une quantité de 0,2 à 4,0 % en poids de la composition,
B. hémisqualane, en une quantité d'au moins 10,0 % en poids de la composition,
C. un émulsifiant, et
D. une huile végétale, de préférence de l'huile de tournesol.
